# EUROPEAN PATENT SPECIFICATION

(11) **EP 4 140 406 B1**
(45) Date of publication and mention of the grant of the patent: **15.05.2024**
(21) Application number: 22184649.6
(22) Date of filing: 13.07.2022
(51) Int. Cl.: A61B 5/11, G06F 18/24, G06F 18/2413

(54) **ENHANCED HUMAN ACTIVITY RECOGNITION**
VERBESSERTE ERKENNUNG MENSCHLICHER AKTIVITÄTEN
RECONNAISSANCE AMÉLIORÉE DES ACTIVITÉS HUMAINES

(30) Priority: 27.08.2021 US 202117459781; 13.04.2022 US 202217720156
(43) Date of publication of application: 01.03.2023
(73) Proprietor: STMicroelectronics S.r.l., 20864 Agrate Brianza (MB) (IT)
(72) Inventor: RIVOLTA, Stefano Paolo, 20832 Desio (MB) (IT); MURA, Roberto, 20151 Milano (IT); FERRAINA, Michele, 20151 Milano (IT)
(74) Representative: Studio Torta S.p.A.

(56) References cited:
- WO-A1-2016/166613
- CN-A- 108 236 469
- US-A1- 2007 100 666
- WILMSDORFF JULIAN VON ET AL: "An experimental overview on electric field sensing", JOURNAL OF AMBIENT INTELLIGENCE AND HUMANIZED COMPUTING, SPRINGER BERLIN HEIDELBERG, BERLIN/HEIDELBERG, vol. 10, no. 2, 11 June 2018 (2018-06-11), pages 813-824, XP036699366, ISSN: 1868-5137, DOI: 10.1007/S12652-018-0877-1 [retrieved on 2018-06-11]
- COHN GABE GABECOHN ET AL: "An ultra-low-power human body motion sensor using static electric field sensing", PROCEEDINGS OF THE SIGCHI CONFERENCE ON HUMAN FACTORS IN COMPUTING SYSTEMS, ACM, 2 PENN PLAZA, SUITE 701 NEW YORK NY 10121-0701 USA, 5 September 2012 (2012-09-05), pages 99-102, XP058618460, DOI: 10.1145/2370216.2370233 ISBN: 978-1-4503-2473-1

## Description

The present disclosure is directed to a system and method for detecting human activity.

Human activity recognition is commonly used in portable devices, such as smart phones, wireless headphones, and smart wearable devices, to detect and track activities of a user. For example, human activity recognition may be used to detect whether a user is stationary, walking, travelling in a vehicle, or performing other types of activities. Devices typically utilize motion sensors to perform human activity recognition. Many devices, for instance, include accelerometers and gyroscopes, and detect a user's activity based on acceleration and angular velocity measurements of the device.

It is desirable for device manufacturers to perform human activity recognition with higher class resolution in order to detect additional types of activities. For example, some devices offer solutions with context awareness in addition to human activity recognition. These types of devices, for example, are capable of distinguishing between indoor or outdoor environments, and may perform specific processes, such as indoor and outdoor navigation.

Devices with context awareness typically distinguish between indoor and outdoor environments by utilizing microphones to detect environmental noise, and audio classification methods to classify the environmental noise as an indoor or outdoor environment. Unfortunately, the use of microphones consumes large amounts of power. In addition, audio classification methods are sensitive to where the device is being carried, as a microphone signal will differ between the device being carried in a user's hand or a user's pocket. Consequently, current audio classification methods employ extensive dataset training and complex deep learning algorithms. Due to the high power and processing demands of current context awareness implementations, context awareness are mostly limited to premium devices. Many low cost, budget devices do not have context awareness capabilities.

Therefore, it is an aim of the present invention to provide a device and a method that overcome the drawbacks of the prior art.

WO 2016/166613discloses determining a user activity based on a plurality of baseline each baseline signature corresponding to a type of user activity and having data formed from a first data representing a varying static electric field and a second data representing motion. Data responsive to a varying static electric field is obtained from a first sensor, and data responsive to motion is obtained from a second sensor. The first data is combined with the second data, and the user activity is identified based on a comparison of the combined first and second data with the plurality of baseline signatures.

US 2007/100666 discloses a monitoring system comprising a module having one sensor which could be an electric-field sensor. The module may display output data including both detected and derived data relating to physiological and contextual parameters of the wearer. The system is capable of deriving and predicting the occurrence of a number of physiological and conditional states and events and reporting the same as output data

According to the invention, a device and a method for detecting human activity are provided, according to the attached claims.

In practice, a device is described that detects a human activity with one or more motion sensors, determines whether the device is in an indoor environment or an outdoor environment, and enhances the detected human activity depending on whether the device is in an indoor environment or an outdoor environment. For example, the device validates the detected human activity or refines the detected human activity to a new activity depending on whether the device is in an indoor environment or an outdoor environment.

In contrast to audio-based solutions that utilize microphones to detect an indoor and outdoor environment, the device disclosed herein utilizes one or more electrostatic charge sensors to determine whether the device is in an indoor environment or an outdoor environment. The device disclosed herein consumes significantly less power and has faster processing times compared to devices with audio-based solutions.

The device may also exclude gyroscope data when performing human activity recognition, and instead utilize electrostatic charge variation data in conjunction with acceleration data to perform human activity recognition. The electrostatic charge variation data adds informative information for the human activity recognition, without adding significant complexity to its algorithm. Further, the use of electrostatic charge data instead of gyroscope data reduces memory consumption for hardware implementations and power consumption.

For a better understanding of the invention, reference will now be made by way of example to the accompanying drawings. In the drawings, identical reference numbers identify similar features or elements. The size and relative positions of features in the drawings are not necessarily drawn to scale.
Figure 1 is a block diagram of a device for performing human activity recognition according to an embodiment disclosed herein.
Figure 2 is a diagram of an electrostatic charge sensor that may be used in the device of Figure 1.
Figure 3 is a flow diagram of a method of detecting human activity.
Figure 4 is an electrostatic charge measurement signal in a case where a device is indoors.
Figure 5 is an electrostatic charge measurement signal in a case where a device is outdoors.
Figure 6 is a flow diagram of a method of detecting human activity with an accelerometer and electrostatic charge sensor.

As discussed above, some devices offer solutions with context awareness in addition to human activity recognition. Generally, these devices distinguish between indoor and outdoor environments using audio-based solutions. For example, the device detects environmental noise with microphones, and classifies the environmental noise as an indoor or outdoor environment with audio classification methods. The use of microphones and audio classification methods consumes large amounts of power and has high processing demands.

The present disclosure is directed to a device with enhanced human activity recognition. The device utilizes one or more motion sensors to detect human activities, such as remaining stationary, walking, cycling, and driving; and utilizes one or more electrostatic charge sensors to detect whether the device is in an indoor or outdoor environment. The detected indoor or outdoor environment is then used in conjunction with the detected human activity in order to validate detected human activities and detect additional classes of activities.

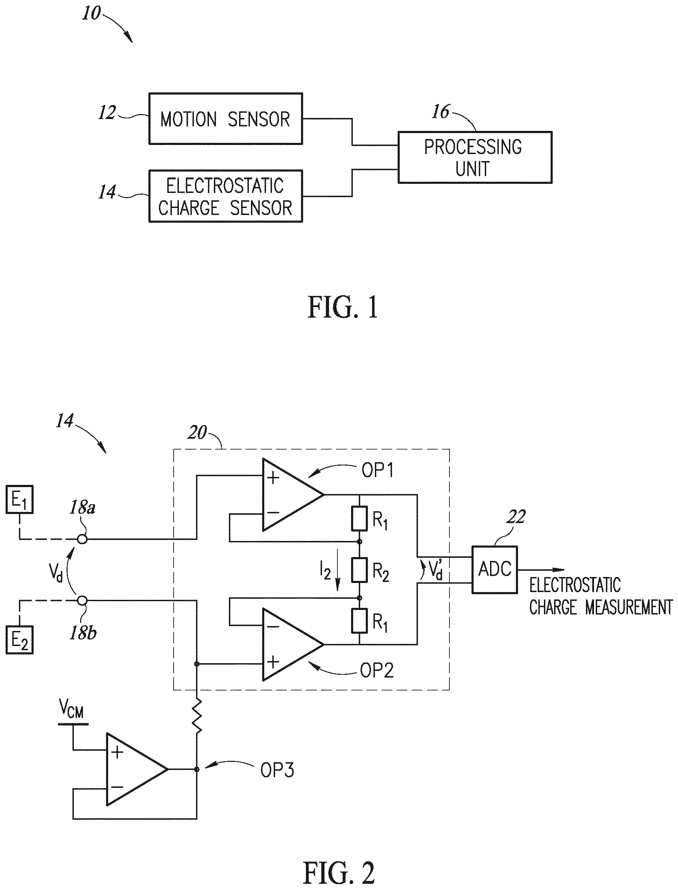

Figure 1 is a block diagram of a device 10. The device 10 is an electronic device that performs human activity recognition. For example, the device 10 may be a smart watch, a fitness tracking device, wireless headphones, a laptop computer, a tablet, or a cellular phone. The device includes a motion sensor 12, an electrostatic charge sensor 14, and a processing unit 16.

The motion sensor 12 is configured to measure a motion of the device 10, and generate a motion measurement that indicates the measured motion. For example, the motion measurement is in the form of an electrical signal (*e*.*g*., voltage or current signal) that is proportional to the measured motion.

The motion sensor 12 is any type of sensor that detects movement of the device 10. For example, the motion sensor 12 may be an accelerometer that measures acceleration along at least one axis (*e*.*g*., a 3-axis accelerometer that measures acceleration along three different axes), or a gyroscope that measures angular velocity along at least one axis (*e.g*., a 3-axis gyroscope that measures angular velocity along three different axes).

Although a single motion sensor 12 is shown in Figure 1, the device 10 may include any number of motion sensors. For instance, the device 10 may include a first motion sensor that is an accelerometer, and a second motion sensor that is a gyroscope.

In the alternative, the motion sensor 12 is a combination sensor that includes both an accelerometer and a gyroscope, where the motion sensor 12 measures both acceleration and angular velocity. Operation of the motion sensor 12 will be discussed in further detail below.

The electrostatic charge sensor 14 is configured to measure an electrostatic charge of a surrounding environment, and generate an electrostatic charge measurement that indicates the measured electrostatic charge. For example, the electrostatic charge measurement is in the form of an electrical signal (*e*.*g*., voltage or current signal) that is proportional to the measured electrostatic charge.

The electrostatic charge sensor 14 includes one or more electrodes that detect electrostatic charge in a surrounding environment, and electrical components (*e*.*g*., resistors, capacitors, amplifiers, etc.) that measure the electrostatic charge detected by the single electrode. Figure 2 is a diagram of an electrostatic charge sensor 14 that may be used in the device 10.

The electrostatic charge sensor 14 includes a pair of input terminals 18a, 18b, coupled to input electrodes E₁, E₂, respectively. Each of the electrodes E₁, E₂ is made of conductive material and, possibly, coated with an insulating layer.

The geometry of the electrodes E₁, E₂ determines the sensitivity and directivity of the electrostatic charge sensor 14. The sensitivity is proportional to the surface area of the electrodes E₁, E₂. The shape of the electrodes E₁, E₂ and their positioning in space affects the directivity of the electrostatic charge sensor 14. For example, the electrodes E₁, E₂ are square in shape, with sides equal to about 2-10 cm (*e.g*., 5 cm).

The electrodes E₁, E₂ may be positioned inside of the device 10. In the alternative, the electrodes E₁, E₂ are positioned on one or more exposed surfaces of the device 10 such that the electrodes E₁, E₂ are exposed to a surrounding environment. For example, the electrodes E₁, E₂ may be positioned on different side surfaces of the device 10.

The pair of input terminals 18a, 18b receive, from the respective electrodes E₁, E₂, an input voltage V_{d} (a differential signal), and supply the input voltage V_{d} to an instrumentation amplifier 20. As will be discussed in further detail below, a presence of an indoor alternating current (AC) power line generates a variation of electrostatic charge which, in turn, after having been detected by the electrodes E₁, E₂, generates the input voltage V_{d}.

The instrumentation amplifier 20 includes operational amplifiers OP1, OP2 and a biasing stage (buffer) OP3. The biasing stage OP3 biases the instrumentation amplifier 20 to a common mode voltage V_{CM}.

An inverting terminal of the amplifier OP1 is electrically connected to an inverting terminal of the amplifier OP2 through a resistor R₂ across which there is a voltage equal to the input voltage V_{d}. Therefore, a current equal to I₂=V_{d}/R₂ will flow through this resistor R₂. The current I₂ does not come from the input terminals of the operational amplifiers OP1, OP2, and, therefore, runs through two resistors R₁ connected between the outputs of the operational amplifiers OP1, OP2, in series with the resistor R₂. The current I₂, which runs through the series of the three resistors R₁-R₂-R₁, produces a differential output voltage V_{d}', which is given by V_{d}'=(2R₁+R₂)I₂=(2R₁+R₂)V_{d}/R₂. The overall gain of the circuit of Figure 2 is Ad=V_{d}'/V_{d}=(2R₁+R₂)/R₂=1+2R₁/R₂. The differential gain depends on the value of the resistor R₂ and may therefore be modified by acting on the resistor R₂.

The differential output voltage V_{d}', therefore being proportional to the potential V_{d} between the input terminals 18a, 18b, is input to an analog-to-digital converter (ADC) 22, which outputs a charge variation signal. The charge variation signal is, for example, a high-resolution digital stream (*e*.*g*., 16 bits or 24 bits). The charge variation signal is an electrostatic charge measurement of an electrostatic charge in a surrounding environment.

In the alternative, the instrumentation amplifier 20 is omitted, so that the ADC 22 receives the differential output voltage V_{d} between the electrodes E₁, E₂ and samples the differential output voltage V_{d} directly. However, the ADC 22 may be omitted, and the charge variation signal is the differential output voltage V_{d}.

In Figure 2, the electrostatic charge sensor 14 includes two electrodes. However, the electrostatic charge sensor 14 may include any number of electrodes. For example, the electrostatic charge sensor 14 may include a single electrode that detects electrostatic charge in a surrounding environment, and electrical components (*e*.*g*., resistors, capacitors, amplifiers, etc.) that measure the electrostatic charge detected by the single electrode.

Although a single electrostatic charge sensor 14 is shown in Figure 1, the device 10 may include any number of electrostatic charge sensors. For example, the device 10 may include a first electrostatic charge sensor having two electrodes, and a second electrostatic charge sensor having another two electrodes.

Returning to Figure 1, the processing unit 16 is communicatively coupled to the motion sensor 12 and the electrostatic charge sensor 14. The processing unit 16 is configured to receive motion measurements from the motion sensor 12 and electrostatic charge measurements from the electrostatic charge sensor 14, and detect or recognize human activity based on the motion measurements and electrostatic charge measurements. The processing unit 16 may be a processor, controller, signal processor, or any other type of processing unit.

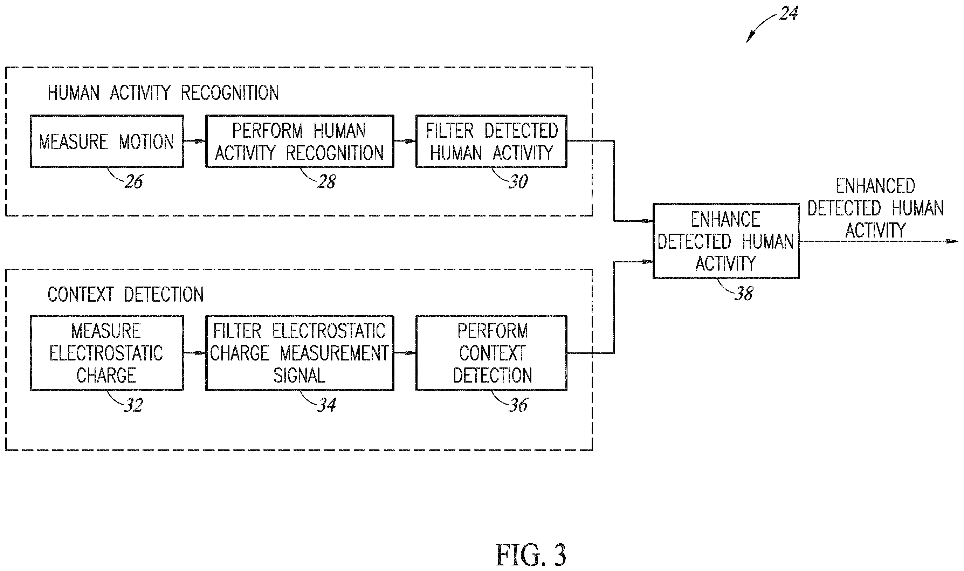

Figure 3 is a flow diagram of a method 24 of detecting human activity that may be performed by the device 10 discussed above. Human activity recognition is performed in blocks 26, 28, and 30; and context detection is performed in blocks 32, 34, and 36.

Turning first to the human activity recognition in blocks 26, 28, and 30, the device 10, more specifically the processing unit 16, performs human activity recognition to classify motion measurements as the stationary state, the walking state, the cycling state, or the driving state. Other types of activities may also be detected.

In block 26, the motion sensor 12 measures motions of the device 10, and generates a motion measurement signal that indicates the measured motions. For example, the device 10 may include a buffer to store a plurality of motion measurements, and generate a motion measurement signal using the plurality of motion measurements when the buffer is full. As discussed above with respect to Figure 1, the motion measurements may be acceleration measurements, angular velocity measurements, another type of motion measurement, or a combination thereof.

In block 28, the processing unit 16 performs human activity recognition based on the motion measurement signal generated in block 26. The human activity recognition determines whether a user of the device 10 is in one of the following states: a stationary state, a walking state, a cycling state, and a driving state.

In the stationary state, the user is remaining still. In the walking state, the user is currently walking either indoors (*e*.*g*., on a treadmill) or outdoors. The walking state may also include the user running either indoors or outdoors. In the cycling state, the user is currently bicycling either indoors on a stationary bicycle or outdoors. In the driving state, the user is currently driving in a vehicle.

The processing unit 16 classifies motion measurements as the stationary state, the walking state, the cycling state, or the driving state using machine learning techniques, such as a decision tree, a neural network, and a support vector machine. For example, the processing unit 16 computes a set of features to match current motion measurements to motion measurements expected for one of a plurality of pre-defined classes of targeted activities of interest. Such an approach utilizes labeled training data for each of the activities desired to be recognized in order to generate the classifier. Moreover, deep neural network models may also be used and implemented for human activity recognition. Deep neural network models are capable of performing automatic feature learning from the raw sensor data. Statistical models (*e*.*g*., Markov models and/or the like) can also be used.

The processing unit 16 may extract features from the motion measurement signal. For example, the processing unit 16 determines at least one of the following calculations to characterize the motion measurement signal: an energy calculation (*e*.*g*., a total energy of the motion measurement signal in a period of time), a variance calculation (*e*.*g*., a variance of the motion measurement signal in a period of time), a zero crossing calculation (*e*.*g*., a number of times the motion measurement signal crosses zero in a period of time), a peak-to-peak calculation (*e*.*g*., a difference between the maximum amplitude and the minimum amplitude of the motion measurement signal in a period of time), a peak count calculation (*e*.*g*., a total number of peaks in the motion measurement signal in a period of time), an absolute mean calculation (*e*.*g*., an absolute mean of the electrostatic charge measurement signal in a period of time), a maximum calculation (*e*.*g*., a maximum of the electrostatic charge measurement signal in a period of time), or a minimum calculation (*e*.*g*., a minimum of the motion measurement signal in a period of time). Other types of calculations are also possible. The features may be extracted within a time window of the motion measurement signal. For example, the features may be calculated within a 2.5 second time window. The time window is, for example, defined based on a minimum number of motion measurements to ensure proper human activity recognition.

The processing unit 16 then classifies the motion measurement signal as the stationary state, the walking state, the cycling state, or the driving state based on the extracted features. A decision tree, such as the one in the following table, may be used to classify the motion measurement signal as the stationary state, the walking state, the cycling state, or the driving state. Other machine learning techniques are also possible. In the following decision tree, the motion measurement signal is an acceleration measurement signal along at least one axis.

| **Condition** | **State** |
|---|---|
| First Peak Count Calculation ≤ First Peak Count Threshold AND | Stationary |
| First Variance Calculation ≤ First Variance Threshold | |
| First Peak Count Calculation ≤ First Peak Count Threshold AND | Stationary |
| First Variance Calculation > First Variance Threshold AND | |
| First Zero Crossing Calculation ≤ First Zero Crossing Threshold AND | |
| First Minimum Calculation ≤ First Minimum Threshold | |
| First Peak Count Calculation ≤ First Peak Count Threshold AND | Walking |
| First Variance Calculation > First Variance Threshold AND | |
| First Zero Crossing Calculation ≤ First Zero Crossing Threshold AND | |
| First Minimum Calculation > First Minimum Threshold | |
| First Peak Count Calculation ≤ First Peak Count Threshold AND | Walking |
| First Variance Calculation > First Variance Threshold AND | |
| First Zero Crossing Calculation > First Zero Crossing Threshold AND | |
| Second Zero Crossing Calculation ≤ Second Zero Crossing Threshold AND | |
| Second Peak Count Calculation ≤ Second Peak Count Threshold | |
| First Peak Count Calculation ≤ First Peak Count Threshold AND | Stationary |
| First Variance Calculation > First Variance Threshold AND | |
| First Zero Crossing Calculation > First Zero Crossing Threshold AND | |
| Second Zero Crossing Calculation ≤ Second Zero Crossing Threshold AND | |
| Second Peak Count Calculation > Second Peak Count Threshold AND | |
| Second Variance Calculation ≤ Second Variance Threshold AND | |
| Third Peak Count Calculation ≤ Third Peak Count Threshold AND | |
| Second Minimum Calculation ≤ Second Minimum Threshold | |
| First Peak Count Calculation ≤ First Peak Count Threshold AND | Walking |
| First Variance Calculation > First Variance Threshold AND | |
| First Zero Crossing Calculation > First Zero Crossing Threshold AND | |
| Second Zero Crossing Calculation ≤ Second Zero Crossing Threshold AND | |
| Second Peak Count Calculation > Second Peak Count Threshold AND | |
| Second Variance Calculation ≤ Second Variance Threshold AND | |
| Third Peak Count Calculation ≤ Third Peak Count Threshold AND | |
| Second Minimum Calculation > Second Minimum Threshold | |
| First Peak Count Calculation ≤ First Peak Count Threshold AND | Stationary |
| First Variance Calculation > First Variance Threshold AND | |
| First Zero Crossing Calculation > First Zero Crossing Threshold AND | |
| Second Zero Crossing Calculation ≤ Second Zero Crossing Threshold AND | |
| Second Peak Count Calculation > Second Peak Count Threshold AND | |
| Second Variance Calculation ≤ Second Variance Threshold AND | |
| Third Peak Count Calculation > Third Peak Count Threshold AND | |
| Third Variance Calculation ≤ Third Variance Threshold | |
| First Peak Count Calculation ≤ First Peak Count Threshold AND | Stationary |
| First Variance Calculation > First Variance Threshold AND | |
| First Zero Crossing Calculation > First Zero Crossing Threshold AND | |
| Second Zero Crossing Calculation ≤ Second Zero Crossing Threshold AND | |
| Second Peak Count Calculation > Second Peak Count Threshold AND | |
| Second Variance Calculation ≤ Second Variance Threshold AND | |
| Third Peak Count Calculation > Third Peak Count Threshold AND | |
| Third Variance Calculation > Third Variance Threshold AND | |
| Third Minimum Calculation ≤ Third Minimum Threshold | |
| First Peak Count Calculation ≤ First Peak Count Threshold AND | Walking |
| First Variance Calculation > First Variance Threshold AND | |
| First Zero Crossing Calculation > First Zero Crossing Threshold AND | |
| Second Zero Crossing Calculation ≤ Second Zero Crossing Threshold AND | |
| Second Peak Count Calculation > Second Peak Count Threshold AND | |
| Second Variance Calculation ≤ Second Variance Threshold AND | |
| Third Peak Count Calculation > Third Peak Count Threshold AND | |
| Third Variance Calculation > Third Variance Threshold AND | |
| Third Minimum Calculation > Third Minimum Threshold | |
| First Peak Count Calculation ≤ First Peak Count Threshold AND | Walking |
| First Variance Calculation > First Variance Threshold AND | |
| First Zero Crossing Calculation > First Zero Crossing Threshold AND | |
| Second Zero Crossing Calculation ≤ Second Zero Crossing Threshold AND | |
| Second Peak Count Calculation > Second Peak Count Threshold AND | |
| Second Variance Calculation > Second Variance Threshold | |
| First Peak Count Calculation ≤ First Peak Count Threshold AND | Stationary |
| First Variance Calculation > First Variance Threshold AND | |
| First Zero Crossing Calculation > First Zero Crossing Threshold AND | |
| Second Zero Crossing Calculation > Second Zero Crossing Threshold | |
| First Peak Count Calculation > First Peak Count Threshold AND | Stationary |
| First Peak-To-Peak Calculation ≤ First Peak-To-Peak Threshold AND | |
| Second Peak-To-Peak Calculation ≤ Second Peak-To-Peak Threshold | |
| First Peak Count Calculation > First Peak Count Threshold AND | Stationary |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation ≤ Third Zero Crossing Threshold AND | |
| Fourth Peak Count Calculation ≤ Fourth Peak Count Threshold | |
| First Peak Count Calculation > First Peak Count Threshold AND | Stationary |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation ≤ Third Zero Crossing Threshold AND | |
| Fourth Peak Count Calculation > Fourth Peak Count Threshold AND | |
| Fourth Zero Crossing Calculation ≤ Fourth Zero Crossing Threshold | |
| First Peak Count Calculation > First Peak Count Threshold AND | Stationary |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation ≤ Third Zero Crossing Threshold AND | |
| Fourth Peak Count Calculation > Fourth Peak Count Threshold AND | |
| Fourth Zero Crossing Calculation > Fourth Zero Crossing Threshold AND | |
| Fourth Variance Calculation ≤ Fourth Variance Threshold AND | |
| Fifth Variance Calculation ≤ Fifth Variance Threshold | |
| First Peak Count Calculation > First Peak Count Threshold AND | Driving |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation ≤ Third Zero Crossing Threshold AND | |
| Fourth Peak Count Calculation > Fourth Peak Count Threshold AND | |
| Fourth Zero Crossing Calculation > Fourth Zero Crossing Threshold AND | |
| Fourth Variance Calculation ≤ Fourth Variance Threshold AND | |
| Fifth Variance Calculation > Fifth Variance Threshold | |
| First Peak Count Calculation > First Peak Count Threshold AND | Stationary |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation ≤ Third Zero Crossing Threshold AND | |
| Fourth Peak Count Calculation > Fourth Peak Count Threshold AND | |
| Fourth Zero Crossing Calculation > Fourth Zero Crossing Threshold AND | |
| Fourth Variance Calculation > Fourth Variance Threshold AND | |
| Fifth Peak Count Calculation ≤ Fifth Peak Count Threshold | |
| First Peak Count Calculation > First Peak Count Threshold AND | Driving |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation ≤ Third Zero Crossing Threshold AND | |
| Fourth Peak Count Calculation > Fourth Peak Count Threshold AND | |
| Fourth Zero Crossing Calculation > Fourth Zero Crossing Threshold AND | |
| Fourth Variance Calculation > Fourth Variance Threshold AND | |
| Fifth Peak Count Calculation > Fifth Peak Count Threshold | |
| First Peak Count Calculation > First Peak Count Threshold AND | Stationary |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation > Third Zero Crossing Threshold AND | |
| Sixth Variance Calculation ≤ Sixth Variance Threshold AND | |
| Seventh Variance Calculation ≤ Seventh Variance Threshold AND | |
| Sixth Peak Count Calculation ≤ Sixth Peak Count Threshold AND | |
| Third Peak-To-Peak Calculation ≤ Third Peak-To-Peak Threshold | |
| First Peak Count Calculation > First Peak Count Threshold AND | Stationary |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation > Third Zero Crossing Threshold AND | |
| Sixth Variance Calculation ≤ Sixth Variance Threshold AND | |
| Seventh Variance Calculation ≤ Seventh Variance Threshold AND | |
| Sixth Peak Count Calculation ≤ Sixth Peak Count Threshold AND | |
| Third Peak-To-Peak Calculation > Third Peak-To-Peak Threshold AND | |
| Seventh Peak Count Calculation ≤ Seventh Peak Count Threshold | |
| First Peak Count Calculation > First Peak Count Threshold AND | Driving |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation > Third Zero Crossing Threshold AND | |
| Sixth Variance Calculation ≤ Sixth Variance Threshold AND | |
| Seventh Variance Calculation ≤ Seventh Variance Threshold AND | |
| Sixth Peak Count Calculation ≤ Sixth Peak Count Threshold AND | |
| Third Peak-To-Peak Calculation > Third Peak-To-Peak Threshold AND | |
| Seventh Peak Count Calculation > Seventh Peak Count Threshold | |
| First Peak Count Calculation > First Peak Count Threshold AND | Driving |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation > Third Zero Crossing Threshold AND | |
| Sixth Variance Calculation ≤ Sixth Variance Threshold AND | |
| Seventh Variance Calculation ≤ Seventh Variance Threshold AND | |
| Sixth Peak Count Calculation > Sixth Peak Count Threshold | |
| First Peak Count Calculation > First Peak Count Threshold AND | Driving |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation > Third Zero Crossing Threshold AND | |
| Sixth Variance Calculation ≤ Sixth Variance Threshold AND | |
| Seventh Variance Calculation > Seventh Variance Threshold | |
| First Peak Count Calculation > First Peak Count Threshold AND | Stationary |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation > Third Zero Crossing Threshold AND | |
| Sixth Variance Calculation > Sixth Variance Threshold AND | |
| Eighth Variance Calculation ≤ Eighth Variance Threshold AND | |
| Eighth Peak Count Calculation ≤ Eighth Peak Count Threshold AND | |
| Ninth Peak Count Calculation ≤ Ninth Peak Count Threshold | |
| First Peak Count Calculation > First Peak Count Threshold AND | Driving |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation > Third Zero Crossing Threshold AND | |
| Sixth Variance Calculation > Sixth Variance Threshold AND | |
| Eighth Variance Calculation ≤ Eighth Variance Threshold AND | |
| Eighth Peak Count Calculation ≤ Eighth Peak Count Threshold AND | |
| Ninth Peak Count Calculation > Ninth Peak Count Threshold AND | |
| Fourth Peak-To-Peak Calculation ≤ Fourth Peak-To-Peak Threshold | |
| First Peak Count Calculation > First Peak Count Threshold AND | Stationary |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation > Third Zero Crossing Threshold AND | |
| Sixth Variance Calculation > Sixth Variance Threshold AND | |
| Eighth Variance Calculation ≤ Eighth Variance Threshold AND | |
| Eighth Peak Count Calculation ≤ Eighth Peak Count Threshold AND | |
| Ninth Peak Count Calculation > Ninth Peak Count Threshold AND | |
| Fourth Peak-To-Peak Calculation > Fourth Peak-To-Peak Threshold AND | |
| Tenth Peak Count Calculation ≤ Tenth Peak Count | |
| First Peak Count Calculation > First Peak Count Threshold AND | Driving |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation > Third Zero Crossing Threshold AND | |
| Sixth Variance Calculation > Sixth Variance Threshold AND | |
| Eighth Variance Calculation ≤ Eighth Variance Threshold AND | |
| Eighth Peak Count Calculation ≤ Eighth Peak Count Threshold AND | |
| Ninth Peak Count Calculation > Ninth Peak Count Threshold AND | |
| Fourth Peak-To-Peak Calculation > Fourth Peak-To-Peak Threshold AND | |
| Tenth Peak Count Calculation > Tenth Peak Count | |
| First Peak Count Calculation > First Peak Count Threshold AND | Driving |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation > Third Zero Crossing Threshold AND | |
| Sixth Variance Calculation > Sixth Variance Threshold AND | |
| Eighth Variance Calculation ≤ Eighth Variance Threshold AND | |
| Eighth Peak Count Calculation > Eighth Peak Count Threshold | |
| First Peak Count Calculation > First Peak Count Threshold AND | Stationary |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation > Third Zero Crossing Threshold AND | |
| Sixth Variance Calculation > Sixth Variance Threshold AND | |
| Eighth Variance Calculation > Eighth Variance Threshold AND | |
| Fifth Zero Crossing Calculation ≤ Fifth Zero Crossing Threshold | |
| First Peak Count Calculation > First Peak Count Threshold AND | Cycling |
| First Peak-To-Peak Calculation ≤ First Peak-To-Peak Threshold AND | |
| Second Peak-To-Peak Calculation > Second Peak-To-Peak Threshold AND | |
| Third Zero Crossing Calculation > Third Zero Crossing Threshold AND | |
| Sixth Variance Calculation > Sixth Variance Threshold AND | |
| Eighth Variance Calculation > Eighth Variance Threshold AND | |
| Fifth Zero Crossing Calculation > Fifth Zero Crossing Threshold | |
| First Peak Count Calculation > First Peak Count Threshold AND | Stationary |
| First Peak-To-Peak Calculation > First Peak-To-Peak Threshold AND | |
| Eleventh Peak Count Calculation ≤ Eleventh Peak Count Threshold AND | |
| Sixth Zero Crossing Calculation ≤ Sixth Zero Crossing Threshold | |
| First Peak Count Calculation > First Peak Count Threshold AND | Cycling |
| First Peak-To-Peak Calculation > First Peak-To-Peak Threshold AND | |
| Eleventh Peak Count Calculation ≤ Eleventh Peak Count Threshold AND | |
| Sixth Zero Crossing Calculation > Sixth Zero Crossing Threshold | |
| First Peak Count Calculation > First Peak Count Threshold AND | Cycling |
| First Peak-To-Peak Calculation > First Peak-To-Peak Threshold AND | |
| Eleventh Peak Count Calculation > Eleventh Peak Count Threshold | |

In the decision tree above, the first zero crossing threshold is less than the second zero crossing threshold, the third zero crossing threshold is greater than the fourth zero crossing threshold, and the third zero crossing threshold is less than the fifth zero crossing threshold. The first peak count threshold is greater than the second peak count threshold, the second peak count threshold is less than the third peak count threshold, the first peak count threshold is less than the fourth peak count threshold, the fourth peak count threshold is less than the fifth peak count threshold, the first peak count threshold is less than the sixth peak count threshold, the sixth peak count threshold is greater than the seventh peak count threshold, the first peak count threshold is less than the eighth peak count threshold, the eighth peak count threshold is greater than the ninth peak count threshold, the ninth peak count threshold is less than the tenth peak count threshold, and the first peak count threshold is less than the eleventh peak count threshold.

In addition, in the decision tree above, the first variance threshold is less than the second variance threshold, the second variance threshold is greater than the third variance threshold, the fourth variance threshold is greater than the fifth variance threshold, the sixth variance threshold is greater than the seventh variance threshold, and the sixth variance threshold is less than the eighth variance threshold.

In addition, in the decision tree above, the first minimum threshold is greater than the second minimum threshold, and the second minimum threshold is less than the third minimum threshold.

In addition, in the decision tree above, the first peak-to-peak threshold is greater than the second peak-to-peak threshold, the second peak-to-peak threshold is greater than the third peak-to-peak threshold, and the second peak-to-peak threshold is less than the fourth peak-to-peak threshold.

In block 30, the processing unit 16 filters the classifications of the motion measurement signal in block 28 in order to eliminate or reduce false positives. Stated differently, the processing unit 16 eliminates classifications of the motions measurement signal as the stationary state, the walking state, the cycling state, or the driving state that are likely to be incorrect.

The processing unit 16 may reduce false classifications by maintaining a count value for each possible state (*i.e*., for each of the stationary state, the walking state, the cycling state, and the driving state). The count value is a total number of times the processing unit 16 classified the motion measurement signal as a particular state (*e*.*g*., the stationary state, the walking state, the cycling state, or the driving state). When the total number is equal to or greater than a threshold count value, the processing unit 16 determines that the motion measurement signal is in the state.

It is noted that block 30 may be removed from the method 24 (*i.e*., not performed) to reduce latency of the human activity recognition.

As will be discussed in further detail below, in block 38, the state detected in block 30 (or block 28 in the case where block 30 is removed) is subsequently enhanced based on context detection results.

Now turning to the context detection in blocks 32, 34, and 36, the device 10, more specifically the processing unit 16, performs context detection to detect whether the device 10 is in an indoor or outdoor environment. The processing unit 16 determines whether the device 10 is an indoor or outdoor environment by detecting an indoor AC power line that provides power to the home or building. The processing unit 16 determines the device 10 is in an indoor environment in response to detecting an indoor AC power line, and determines the device 10 is in an outdoor environment in response to detecting that an indoor AC power line is not present.

In block 32, the electrostatic charge sensor 14 measures an electrostatic charge of a surrounding environment, and generates an electrostatic charge measurement signal that indicates the measured electrostatic charges. For example, the device 10 includes a buffer to store a plurality of electrostatic charge measurements, and generates an electrostatic charge measurement signal using the plurality of electrostatic charge measurements when the buffer is full. The electrostatic charge sensor 14 may measure electrostatic charge continuously or periodically (*e*.*g*., every 10 seconds).

In block 34, the processing unit 16 filters the electrostatic charge measurement signal to remove unnecessary electrostatic charge measurements. To this end, a high pass filter may be applied to the electrostatic charge measurement signal. A cutoff frequency of the high pass filter is selected based on the frequency of the AC power line being detected. For example, the cutoff frequency may be set to 40 hertz when a 50 hertz and a 60 hertz AC power line is being detected. Other types of filters, such as a low pass filter and band pass filter, may also be applied to the electrostatic charge measurement signal.

It is noted that block 34 may be removed from the method 24 (*i.e*., not performed).

In block 36, the processing unit 16 performs context detection based on the filtered electrostatic charge signal generated in block 34 (or the electrostatic charge signal generated in block 32 in the case where block 34 is removed). As mentioned above, the context detection detects whether the device 10 is in an indoor or outdoor environment. Context detection is performed using either a frequency approach or a time domain approach.

In the frequency approach, the processing unit 16 converts the electrostatic measurement signal to the frequency domain by applying, for example, a fast Fourier transform to the electrostatic measurements. Other techniques for converting the electrostatic measurements to the frequency domain may also be used.

The processing unit 16 then performs tone detection to detect whether electrostatic charge signal includes a peak corresponding to a frequency of an indoor AC power line. When the device 10 is indoors and near an AC power line, the processing unit 16 detects an electrostatic charge with a frequency corresponding to a frequency of the AC power line. For example, in a case where the device 10 is in a house with an AC power line having a frequency of 60 hertz, the electrostatic charge sensor 14 detects an electrostatic charge peak or tone at 60 hertz. Conversely, in a case where the device 10 is outdoors and is not in proximity to an AC power line, the processing unit 16 does not detect an electrostatic charge peak at 60 hertz, or detects an electrostatic charge peak at 60 hertz that is substantially smaller than the 60 hertz electrostatic charge peak detected in the case where the device 10 is in the house with the AC power line having a frequency of 60 hertz.

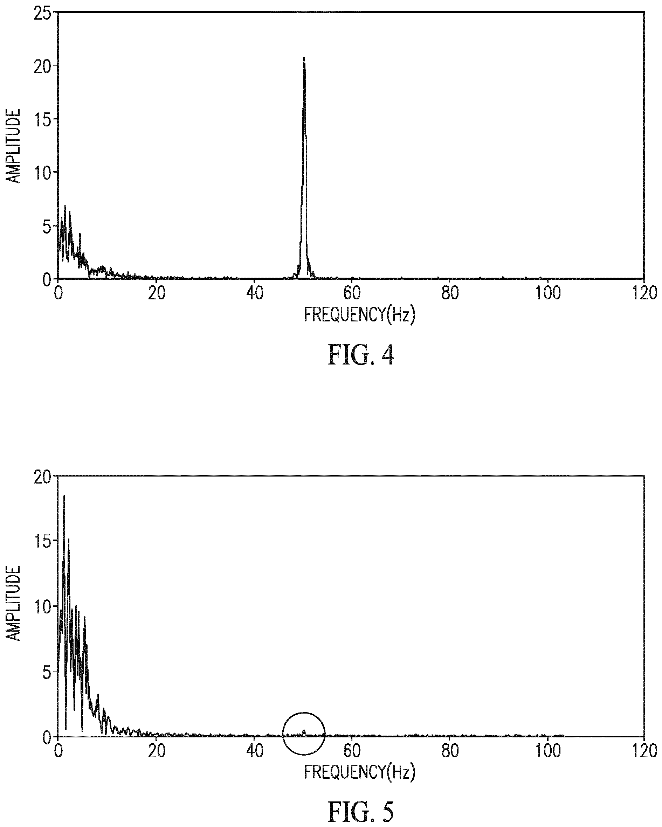

For example, Figure 4 is an electrostatic charge measurement signal in a case where the device 10 is indoors, and Figure 5 is an electrostatic charge measurement signal in a case where the device 10 is outdoors. In the example shown in Figure 4, the indoor AC power line has a frequency of 50 hertz. Detection of AC power lines with other frequencies (*e*.*g*., 60 hertz) is also possible.

In Figures 4 and 5, the vertical axes are amplitudes of the electrostatic charge measurement signals, and the horizontal axes are frequency axes. The amplitudes are outputs from an ADC, and the frequency axes are in hertz. Other amplitude and frequency units are also possible.

As can be seen in Figure 4, the electrostatic charge sensor 14 detects an electrostatic charge peak or tone at approximately 50 hertz. Thus, in this example, the device 10 is in the presence of a 50 hertz AC power line, and the processing unit 16 determines the device 10 is indoors. In contrast, in Figure 5, the electrostatic charge sensor 14 detects an electrostatic charge peak at approximately 50 hertz (in the encircled area in Figure 5) that is substantially smaller than the 50 hertz electrostatic charge peak detected in Figure 4. Thus, in this example, the device 10 is not in the presence of a 50 hertz AC power line, and the processing unit 16 determines the device 10 is outdoors.

Accordingly, in the frequency approach, the processing unit 16 determines whether the device 10 is indoors or outdoors based on whether or not the electrostatic charge signal includes a peak at a frequency of an indoor AC power line.

In the time domain approach, the processing unit 16 classifies electrostatic charge measurements in the time domain as an indoor state or an outdoor state using either deterministic or machine learning techniques, such as a decision tree, a neural network, and a support vector machine. For example, similar to the human activity recognition in block 28, the processing unit 16 computes a set of features to match current electrostatic charge measurements to electrostatic charge measurements expected for the indoor state and the outdoor state. Such an approach utilizes labeled training data for each of the indoor state and the outdoor state in order to generate the classifier. Moreover, deep neural network models may also be used and implemented for indoor and outdoor detection. Deep neural network models are capable of performing automatic feature learning from the raw sensor data and out-perform models fit on hand-crafted domain-specific features. Statistical models (*e*.*g*., Markov models and/or the like) can also be used.

The processing unit 16 may extract features from the electrostatic charge measurement signal. For example, the processing unit 16 determines at least one of the following calculations, which were also discussed above with respect to block 28, to characterize the electrostatic charge measurement signal: an energy calculation, a variance calculation, a zero crossing calculation, a peak-to-peak calculation, a peak count calculation, an absolute mean calculation, a maximum calculation, or a minimum calculation. Other types of calculations are also possible. For example, the features are extracted within a time window of the electrostatic charge measurement signal. For example, the features may be calculated within a 2.5 second time window. The time window is, for example, defined based on a minimum number of electrostatic charge measurements to ensure proper context detection.

The processing unit 16 then classifies the electrostatic charge measurement signal as the indoor state or the outdoor state based on the extracted features. A decision tree, such as the one in the following table, may be used to classify the electrostatic charge measurement signal as the indoor state or the outdoor state. Other machine learning techniques are also possible.

| **Condition** | **State** |
|---|---|
| First Variance Calculation ≤ First Variance Threshold AND | Outdoor |
| Zero Crossing Calculation ≤ Zero Crossing Threshold | |
| First Variance Calculation ≤ First Variance Threshold AND | Outdoor |
| Zero Crossing Calculation > Zero Crossing Threshold AND | |
| Peak Count Calculation ≤ Peak Count Threshold AND | |
| Second Variance Calculation ≤ Second Variance Threshold | |
| First Variance Calculation ≤ First Variance Threshold AND | Indoor |
| Zero Crossing Calculation > Zero Crossing Threshold AND | |
| Peak Count Calculation ≤ Peak Count Threshold AND | |
| Second Variance Calculation > Second Variance Threshold | |
| First Variance Calculation ≤ First Variance Threshold AND | Indoor |
| Zero Crossing Calculation > Zero Crossing Threshold AND | |
| Peak Count Calculation > Peak Count Threshold AND | |
| Peak-To-Peak Calculation ≤ Peak-To-Peak Threshold | |
| First Variance Calculation ≤ First Variance Threshold AND | Outdoor |
| Zero Crossing Calculation > Zero Crossing Threshold AND | |
| Peak Count Calculation > Peak Count Threshold AND | |
| Peak-To-Peak Calculation > Peak-To-Peak Threshold | |
| First Variance Calculation > First Variance Threshold AND | Indoor |

In the decision tree above, the first variance threshold is greater than the second variance threshold.

In block 38, the state detected in block 30 (or block 28 in the case where block 30 is removed) is enhanced based on the context detected in block 36. Namely, in block 38, the stationary state, the walking state, the cycling state, or the driving state determined in block 30 is refined based on whether the device 10 is determined to be indoors or outdoors in block 36.

In block 38, the processing unit 16 may validate the human activity detected in block 30 (or block 28 in the case where block 30 is removed) by rejecting false state detections (*i*.*e*., incorrectly detected states).

The processing unit 16 detects false state detections based on whether the device 10 is determined to be indoors or outdoors. For example in the event the processing unit 16 determines a user of the device 10 is in the driving state and determines that the device 10 is indoors, the processing unit 16 determines that the detection of the driving state is a false detection as it is highly unlikely a user is driving a vehicle indoors.

The processing unit 16 then switches the driving state to, for example, the stationary state; and outputs the stationary state as the enhanced detected human activity. Conversely, in the event the processing unit 16 determines a user of the device 10 is in the driving state and determines that the device 10 is outdoors, the processing unit 16 determines that the detection of the driving state is a true detection, and outputs the driving state as the enhanced detected human activity.

In block 38, the processing unit 16 may refine or adjust the state detected in block 30 to a new classification. In the event the processing unit 16 determines a user of the device 10 is in the walking state and determines that the device 10 is indoors, the processing unit 16 switches the walking state to an indoor walking state that indicates the user is walking or running indoors, and outputs the indoor walking state as the enhanced detected human activity.

In the event the processing unit 16 determines a user of the device 10 is in the walking state and determines that the device 10 is outdoors, the processing unit 16 switches the walking state to an outdoor walking state that indicates the user is walking or running outside, and outputs the outdoor walking state as the enhanced detected human activity.

In the event the processing unit 16 determines a user of the device 10 is in the cycling state and determines that the device 10 is indoors, the processing unit 16 switches the cycling state to a stationary bicycle state that indicates the user is cycling on a stationary bicycle, and outputs the stationary bicycle state as the enhanced detected human activity.

In the event the processing unit 16 determines a user of the device 10 is in the cycling state and determines that the device 10 is outdoors, the processing unit 16 switches the cycling state to an outdoor cycling state that indicates the user is cycling outside, and outputs the outdoor cycling state as the enhanced detected human activity. Here, motion measurements may be classified as the stationary state, the indoor walking state, the outdoor walking state, the stationary bicycle state, the outdoor cycling state, or the driving state.

In Figure 3, human activity recognition and context detection are performed in parallel with each other, and the detected human activity and the detected context are combined to generate an enhanced detected human activity. Human activity recognition and context detection may also be performed in series. For example, human activity recognition is performed subsequent to context detection, and human activity recognition is adjusted based on the detected context.

In the event the device 10 is determined to be indoors during context detection, human activity recognition is set to detect indoor states and to not detect outdoor states. For example, in the case where machine learning techniques are used for human activity recognition, indoor training data is loaded and used for classification of motion
measurements in block 28 in order to detect the stationary state, the indoor walking state, and the stationary bicycle state.

Conversely, in the event the device 10 is determined to be outdoors during context detection, human activity recognition is set to detect outdoor states and to not detect indoor states. For example, in the case where machine learning techniques are used for human activity recognition, outdoor training data is loaded and used for classification of motion measurements in block 28 in order to detect the stationary state, the outdoor walking state, the outdoor cycling state, and the driving state.

In Figure 3, human activity recognition and context detection are performed separately. For example, the human activity recognition in block 28 is performed using a first decision tree, and the context detection in block 36 is performed using a second, separate decision tree. In the alternative, the human activity recognition and the context detection are combined, and a single decision tree is used to detect whether a user of the device 10 is in the stationary state, the indoor walking state, the outdoor walking state, the cycling state, the driving state, or the stationary bicycle state.

Human activity recognition may be performed based on various motion measurements. For example, as discussed above with respect to Figure 3, the device 10 performs human activity recognition based on the motion measurements made by the motion sensor 12. The motion measurements may include acceleration measurements, angular velocity measurements, another type of motion measurement, or a combination thereof. In some cases, however, gyroscope data is discarded in order to reduce power consumption, particularly in wearable devices where the battery duration is important. Unfortunately, the drop in performance of the human activity recognition without gyroscope data is not always negligible, particularly in cases where the complexity of the algorithm for human activity recognition is limited.

In order to compensate for the loss of gyroscope data, the device 10 utilizes electrostatic charge variation data in conjunction with acceleration data to perform human activity recognition. Stated differently, the device 10, more specifically the processing unit 16, performs human activity recognition based on acceleration measurements generated by an accelerometer of the motion sensor 12, and electrostatic charge variation measurements generated by the electrostatic charge sensor 14.

The electrostatic charge variation data adds informative information for the human activity recognition, without adding significant complexity to its algorithm. The use of electrostatic charge data instead of gyroscope data may even reduce memory consumption for hardware implementations. In addition, power consumption of an electrostatic charge sensor is much less than a gyroscope. An electrostatic charge sensor typically consumes between 10 and 20 microamps during operation, whereas a gyroscope typically consumes between 290 and 310 microamps during operation.

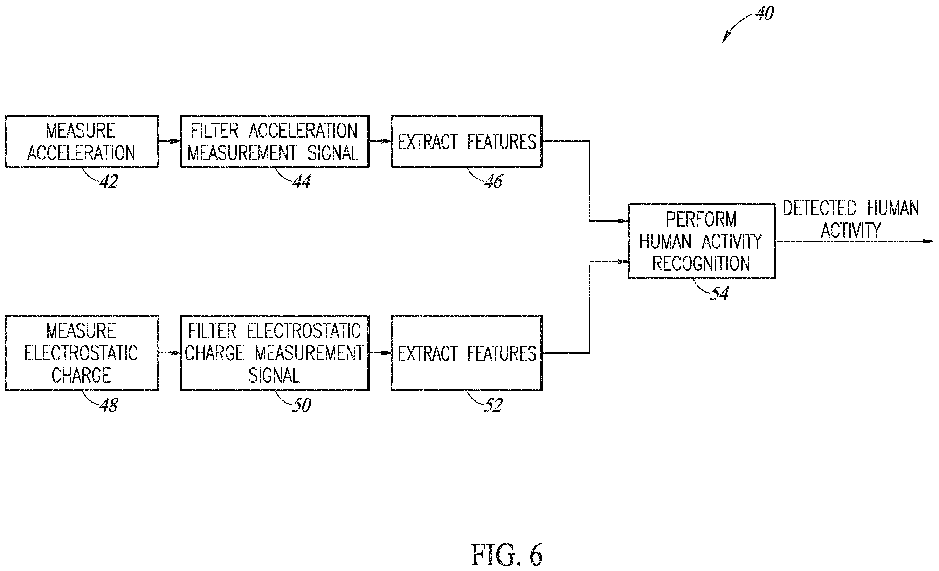

Figure 6 is a flow diagram of a method 40 of detecting human activity with an accelerometer and electrostatic charge sensor.

In block 42, the motion sensor 12, which here is or includes an accelerometer, measures acceleration of the device 10, and generates at least one acceleration measurement signal that indicates the measured acceleration. As noted above, the accelerometer measures acceleration along at least one axis. For example, the accelerometer may be a single axis accelerometer in which case the acceleration measurement signal indicates measured acceleration along the single axis, or a 3-axis accelerometer in which case the motion sensor 12 generates an acceleration measurement signal for each of the three different axes. The device 10 may include a buffer to store a plurality of acceleration measurements, and generate an acceleration measurement signal using the plurality of acceleration measurements when the buffer is full. The method 40 then moves to block 44.

In block 44, the processing unit 16 filters the acceleration measurement signal(s) to remove unnecessary acceleration measurements. For example, a band pass filter is applied to the acceleration measurement signal(s) to enhance accelerations indicative of, for example, a stationary state, a walking state, a running state, a cycling state, and a driving state. For example, the processing unit 16 applies a band pass filter having a lower cutoff frequency of 1 hertz and an upper cutoff frequency of 4 hertz. Other types of filters, such as a low pass filter and a high pass filter, may also be applied to the acceleration measurement signal(s). The method 40 then moves to block 46.

In block 46, the processing unit 16 extracts features from the filtered acceleration measurement signal(s). The extracted features characterize the amplitude and the variability of the filtered acceleration measurement signal(s).

The processing unit 16 determines one or more of the following calculations to characterize the acceleration measurement signal(s): a variance calculation (*e.g*., a variance of the acceleration measurement signal in a period of time), an energy calculation (*e.g*., a total energy of the acceleration measurement signal in a period of time), a zero crossing calculation (*e.g*., a number of times the acceleration measurement signal crosses zero in a period of time), a peak count calculation (*e.g*., a total number of peaks in the acceleration measurement signal in a period of time), a peak-to-peak calculation (*e.g*., a difference between the maximum amplitude and the minimum amplitude of the acceleration measurement signal in a period of time), a minimum calculation (*e.g*., a minimum of the acceleration measurement signal in a period of time), a maximum calculation (*e.g*., a maximum of the acceleration measurement signal in a period of time), and an absolute mean calculation (*e.g*., an absolute mean of the acceleration measurement signal in a period of time). Other types of calculations are also possible.

The features may be extracted within a time window of the acceleration measurement signal(s). For example, the features may be calculated within a 2.5 second time window. The time window is, for example, defined based on a minimum number of acceleration measurements to ensure proper human activity recognition.

The method 40 then moves to block 54. As will be discussed in further detail below, the extracted features determined in block 46 are used for human activity recognition in block 54.

Blocks 48, 50, and 52 are performed concurrently with blocks 42, 44, and 46. In block 48, the electrostatic charge sensor 14 measures electrostatic charge, more specifically electrostatic charge variation, of a surrounding environment; and generates an electrostatic charge measurement signal that indicates the measured electrostatic charges. The device 10 may include a buffer to store a plurality of electrostatic charge measurements, and generate an electrostatic charge measurement signal using the plurality of electrostatic charge measurements when the buffer is full. The electrostatic charge sensor 14 may measure electrostatic charge continuously or periodically (*e.g*., every 10 seconds).

The electrostatic charge sensor 14 includes one or more electrodes that are in physical contact with a user's skin. For example, in a case where the device 10 is a wearable device, the electrodes are positioned on an outer surface of the device 10 such that the electrodes touch the user's skin while the device 10 is being worn by the user. As discussed above, the electrodes of the electrostatic charge sensor 14 detect electrostatic charge, more specifically electrostatic charge variation, in a surrounding environment, and electrical components (*e*.*g*., resistors, capacitors, amplifiers, etc.) of the electrostatic charge sensor 14 measure the electrostatic charge detected by the one or more electrodes.

The method 40 then moves to block 50.

In block 50, the processing unit 16 filters the electrostatic charge measurement signal to remove unnecessary electrostatic charge measurements, such as electrostatic charge variations caused by wind. For example, a band pass filter is applied to the electrostatic charge measurement signal to enhance electrostatic charge indicative of, for example, a stationary state, a walking state, a running state, a cycling state, and a driving state. For example, the processing unit 16 applies a band pass filter having a lower cutoff frequency of 0.01 hertz and an upper cutoff frequency of 5 hertz. Other types of filters, such as a low pass filter and a high pass filter, may also be applied to the electrostatic charge measurement signal. The method 40 then moves to block 52.

In block 52, the processing unit 16 extracts features from the filtered electrostatic charge measurement signal. The extracted features characterize the amplitude and the variability of the filtered electrostatic charge measurement signal.

The processing unit 16 determines one or more of the following calculations to characterize the electrostatic charge measurement signal: a variance calculation (*e*.*g*., a variance of the electrostatic charge measurement signal in a period of time), an energy calculation (*e*.*g*., a total energy of the electrostatic charge measurement signal in a period of time), a zero crossing calculation (*e*.*g*., a number of times the electrostatic charge measurement signal crosses zero in a period of time), a peak count calculation (*e*.*g*., a total number of peaks in the electrostatic charge measurement signal in a period of time), a peak-to-peak calculation (*e*.*g*., a difference between the maximum amplitude and the minimum amplitude of the electrostatic charge measurement signal in a period of time), a minimum calculation (*e*.*g*., a minimum of the electrostatic charge measurement signal in a period of time), a maximum calculation (*e.g*., a maximum of the electrostatic charge measurement signal in a period of time), and an absolute mean calculation (*e.g*., an absolute mean of the electrostatic charge measurement signal in a period of time). Other types of calculations are also possible.

The features may be extracted within a time window of the electrostatic charge measurement signal. For example, the features may be calculated within a 2.5 second time window. The time window is, for example, defined based on a minimum number of electrostatic charge measurements to ensure proper human activity recognition.

The method 40 then moves to block 54.

In block 54, the processing unit 16 performs human activity recognition based on the extracted features from the acceleration measurement signal(s) and the extracted features from the electrostatic charge measurement signal. Here, the human activity recognition determines whether a user of the device 10 is in one of the following states: a stationary state, a walking state, a running state, a cycling state, and a driving state.

In the stationary state, the user is remaining still. In the walking state, the user is currently walking. In the running state, the user is currently running. In the cycling state, the user is currently bicycling. In the driving state, the user is currently driving in a vehicle.

A decision tree, such as the one in the following table, may be used to detect the stationary state, the walking state, the running state, the cycling state, or the driving state. Other machine learning techniques, such as a neural network and a support vector machine, are also possible. In the following decision tree, "XL" refers to a feature extracted from the acceleration measurement signal(s) in block 46, and "EC" refers to a feature extracted from the electrostatic charge measurement signal in block 52.

| **Condition** | **State** |
|---|---|
| First XL Peak-to-Peak Calculation ≤ First XL Peak-to-Peak Threshold AND | Walking |
| First XL Peak Count Calculation ≤ First XL Peak Count Threshold | |
| First XL Peak-to-Peak Calculation ≤ First XL Peak-to-Peak Threshold AND | Driving |
| First XL Peak Count Calculation > First XL Peak Count Threshold AND | |
| First EC Energy Calculation ≤ First EC Energy Threshold AND | |
| First EC Minimum Calculation ≤ First EC Minimum Threshold AND | |
| First XL Zero Crossing Calculation ≤ First EC Zero Crossing Threshold AND | |
| First EC Peak Count Calculation ≤ First EC Peak Count Threshold AND | |
| Second EC Peak Count Calculation ≤ Second EC Peak Count Threshold | |
| First XL Peak-to-Peak Calculation ≤ First XL Peak-to-Peak Threshold AND | Stationary |
| First XL Peak Count Calculation > First XL Peak Count Threshold AND | |
| First EC Energy Calculation ≤ First EC Energy Threshold AND | |
| First EC Minimum Calculation ≤ First EC Minimum Threshold AND | |
| First XL Zero Crossing Calculation ≤ First EC Zero Crossing Threshold AND | |
| First EC Peak Count Calculation ≤ First EC Peak Count Threshold AND | |
| Second EC Peak Count Calculation > Second EC Peak Count Threshold AND | |
| Second XL Peak Count Calculation ≤ Second XL Peak Count Threshold | |
| First XL Peak-to-Peak Calculation ≤ First XL Peak-to-Peak Threshold AND | Driving |
| First XL Peak Count Calculation > First XL Peak Count Threshold AND | |
| First EC Energy Calculation ≤ First EC Energy Threshold AND | |
| First EC Minimum Calculation ≤ First EC Minimum Threshold AND | |
| First XL Zero Crossing Calculation ≤ First EC Zero Crossing Threshold AND | |
| First EC Peak Count Calculation ≤ First EC Peak Count Threshold AND | |
| Second EC Peak Count Calculation > Second EC Peak Count Threshold AND | |
| Second XL Peak Count Calculation > Second XL Peak Count Threshold | |
| First XL Peak-to-Peak Calculation ≤ First XL Peak-to-Peak Threshold AND | Stationary |
| First XL Peak Count Calculation > First XL Peak Count Threshold AND | |
| First EC Energy Calculation ≤ First EC Energy Threshold AND | |
| First EC Minimum Calculation ≤ First EC Minimum Threshold AND | |
| First XL Zero Crossing Calculation ≤ First EC Zero Crossing Threshold AND | |
| First EC Peak Count Calculation > First EC Peak Count Threshold AND | |
| First EC Maximum Calculation ≤ First EC Maximum Threshold | |
| First XL Peak-to-Peak Calculation ≤ First XL Peak-to-Peak Threshold AND | Stationary |
| First XL Peak Count Calculation > First XL Peak Count Threshold AND | |
| First EC Energy Calculation ≤ First EC Energy Threshold AND | |
| First EC Minimum Calculation ≤ First EC Minimum Threshold AND | |
| First XL Zero Crossing Calculation ≤ First EC Zero Crossing Threshold AND | |
| First EC Peak Count Calculation > First EC Peak Count Threshold AND | |
| First EC Maximum Calculation > First EC Maximum Threshold AND | |
| Third XL Peak Count Calculation ≤ Third XL Peak Count Threshold | |
| First XL Peak-to-Peak Calculation ≤ First XL Peak-to-Peak Threshold AND | Driving |
| First XL Peak Count Calculation > First XL Peak Count Threshold AND | |
| First EC Energy Calculation ≤ First EC Energy Threshold AND | |
| First EC Minimum Calculation ≤ First EC Minimum Threshold AND | |
| First XL Zero Crossing Calculation ≤ First EC Zero Crossing Threshold AND | |
| First EC Peak Count Calculation > First EC Peak Count Threshold AND | |
| First EC Maximum Calculation > First EC Maximum Threshold AND | |
| Third XL Peak Count Calculation > Third XL Peak Count Threshold | |
| First XL Peak-to-Peak Calculation ≤ First XL Peak-to-Peak Threshold AND | Cycling |
| First XL Peak Count Calculation > First XL Peak Count Threshold AND | |
| First EC Energy Calculation ≤ First EC Energy Threshold AND | |
| First EC Minimum Calculation ≤ First EC Minimum Threshold AND | |
| First XL Zero Crossing Calculation > First EC Zero Crossing Threshold | |
| First XL Peak-to-Peak Calculation ≤ First XL Peak-to-Peak Threshold AND | Driving |
| First XL Peak Count Calculation > First XL Peak Count Threshold AND | |
| First EC Energy Calculation ≤ First EC Energy Threshold AND | |
| First EC Minimum Calculation > First EC Minimum Threshold AND | |
| Second XL Peak-to-Peak Calculation ≤ Second XL Peak-to-Peak Threshold | |
| First XL Peak-to-Peak Calculation ≤ First XL Peak-to-Peak Threshold AND | Cycling |
| First XL Peak Count Calculation > First XL Peak Count Threshold AND | |
| First EC Energy Calculation ≤ First EC Energy Threshold AND | |
| First EC Minimum Calculation > First EC Minimum Threshold AND | |
| Second XL Peak-to-Peak Calculation > Second XL Peak-to-Peak Threshold | |
| First XL Peak-to-Peak Calculation ≤ First XL Peak-to-Peak Threshold AND | Cycling |
| First XL Peak Count Calculation > First XL Peak Count Threshold AND | |
| First EC Energy Calculation > First EC Energy Threshold | |
| First XL Peak-to-Peak Calculation > First XL Peak-to-Peak Threshold | Running |

In the decision tree above, the first EC peak count threshold is greater than the second EC peak count threshold, the first XL peak count threshold is less than the second XL peak count threshold, the first XL peak count threshold is less than the third XL peak count threshold, and the first XL peak-to-peak calculation is greater than the second XL peak-to-peak calculation.

The detected human activity (*e*.*g*., the stationary state, the walking state, the running state, the cycling state, or the driving state) is then output for further processing. For example, the device 10 changes a state of the device 10 (*e.g*., a displayed screen) based on the detected activity.

The methods 24 and 40 discussed above may be implemented as a software library included in and executed by a microcontroller (*e*.*g*., the processing unit 16) or a communication module (*e.g*., a Bluetooth Low Energy (BLE) module) of the device 10. The methods 24 and 40 may also be implemented as hardware within, for example, a sensor device (*e.g*., a combination sensor that includes the motion sensor 12, the electrostatic charge sensor 14, and the processing unit 16) of the device 10. In this case, the microcontroller may be removed from the device 10.

The description herein provide a device with enhanced human activity recognition. The device detects a human activity and whether the device is in an indoor or outdoor environment, and enhances the detected human activity based on whether the device is in the indoor or outdoor environment. In contrast to audio-based solutions which utilize microphones to detect an indoor and outdoor environment, the device disclosed herein utilizes one or more electrostatic charge sensors to detect an indoor and outdoor environment. The device consumes significantly less power compared to current audio-based solutions. For example, microphones in audio-based solutions typically consume between 120 and 700 micro amps, whereas electrostatic charge sensors typically consume between 10 and 20 micro amps. In addition, the device may also exclude gyroscope data when performing human activity recognition, and instead utilize electrostatic charge variation data in conjunction with acceleration data to perform human activity recognition.

A device may be summarized as including: an accelerometer configured to generate an acceleration measurement; an electrostatic charge sensor configured to generate an electrostatic charge measurement; and a processing unit configured to determine an activity state of a user of the device based on the acceleration measurement and the electrostatic charge measurement, and output the activity state.

The processing unit may be configured to filter the acceleration measurement and the electrostatic charge measurement, and determine the activity state of the user of the device based on the filtered acceleration measurement and the filtered electrostatic charge measurement.

The processing unit may filter the acceleration measurement and the electrostatic charge measurement with a first band pass filter and a second band pass filter, respectively.

The processing unit may be configured to determine at least one first feature of the acceleration measurement and at least one second feature of the electrostatic charge measurement, and determine the activity state of the user of the device based on the at least one first feature and the at least one second feature.

The at least one first feature may include at least one of a variance calculation, an energy calculation, a zero crossing calculation, a peak count calculation, a peak-to-peak calculation, a minimum calculation, a maximum calculation, and an absolute mean calculation, and the at least one second feature may include at least one of a variance calculation, an energy calculation, a zero crossing calculation, a peak count calculation, a peak-to-peak calculation, a minimum calculation, a maximum calculation, and an absolute mean calculation.

The activity state may be a state selected from a group of states including a stationary state that indicates the user is remaining still, a walking state that indicates the user is walking, a running state that indicates the user is running, a cycling state that indicates the user is cycling, and a driving state that indicates the user is driving a vehicle.

The electrostatic charge sensor may include at least one electrode that is positioned on an outer surface of the device such that the at least one electrode physically contacts the user's skin when the device is used by the user.

The processing unit may utilize machine learning to determine the activity state of the user of the device.

The device may further include a combination sensor including the accelerometer, the electrostatic charge sensor, and the processing unit.

A method may be summarized as including: measuring, by an accelerometer, acceleration; filtering, by a processing unit, the acceleration measurement; measuring, by an electrostatic charge sensor, electrostatic charge; filtering, by the processing unit, the electrostatic charge measurement; determining, by the processing unit, an activity state of a user based on the filtered acceleration measurement and the filtered electrostatic charge measurement; and outputting the activity state.

The filtering of the acceleration measurement may include applying a first band pass filter to the acceleration measurement, and the filtering of the electrostatic charge measurement may include applying a second band pass filter to the acceleration measurement.

The method may further include: determining, by the processing unit, at least one first feature of the acceleration measurement and at least one second feature of the electrostatic charge measurement; and determining, by the processing unit, the activity state of the user based on the at least one first feature and the at least one second feature.

The at least one first feature may include at least one of a variance calculation, an energy calculation, a zero crossing calculation, a peak count calculation, a peak-to-peak calculation, a minimum calculation, a maximum calculation, and an absolute mean calculation, and the at least one second feature may include at least one of a variance calculation, an energy calculation, a zero crossing calculation, a peak count calculation, a peak-to-peak calculation, a minimum calculation, a maximum calculation, and an absolute mean calculation.

The activity state may be a state selected from a group of states including a stationary state that indicates the user is remaining still, a walking state that indicates the user is walking, a running state that indicates the user is running, a cycling state that indicates the user is cycling, and a driving state that indicates the user is driving a vehicle.

The method may further include: physically contacting an electrode of the electrostatic charge sensor with the user's skin, the measuring of the electrostatic charge being performed while the electrode is in physical contact with the user's skin.

The determining of the activity state of the user may include using machine learning.

A sensor device may be summarized as including: an accelerometer configured to measure acceleration; an electrostatic charge sensor including at least one electrode, and configured to measure electrostatic charge with the at least one electrode; and a processing unit configured to: filter the measured acceleration; extract at least one first feature from the filtered measured acceleration; filter the measured electrostatic charge; extract at least one second feature from the filtered measured electrostatic charge; determine an activity state of a user based on the at least one first feature and the at least one second feature; and output the activity state.

The electrostatic charge may be measured while the at least one electrode is in physical contact with the user's skin.

The at least one first feature may include at least one of a variance calculation, an energy calculation, a zero crossing calculation, a peak count calculation, a peak-to-peak calculation, a minimum calculation, a maximum calculation, and an absolute mean calculation, and the at least one second feature may include at least one of a variance calculation, an energy calculation, a zero crossing calculation, a peak count calculation, a peak-to-peak calculation, a minimum calculation, a maximum calculation, and an absolute mean calculation.

The activity state may be a state selected from a group of states including a stationary state that indicates the user is remaining still, a walking state that indicates the user is walking, a running state that indicates the user is running, a cycling state that indicates the user is cycling, and a driving state that indicates the user is driving a vehicle.

## Claims

1. A device for detecting human activity, comprising:
a motion sensor (12) configured to generate a motion measurement of the device, the motion measurement detecting human activities, such as remaining stationary, walking, cycling or driving;
an electrostatic charge sensor (14) configured to generate an electrostatic charge measurement of the device, the electrostatic charge measurement detecting whether the device is in an indoor or outdoor environment; and
a processing unit (16) configured to determine an activity state of a user of the device based on the motion measurement and the electrostatic charge measurement, and output the activity state, **characterised in that** the processing unit is
configured to use the detected indoor or outdoor environment in conjunction with the detected human activity to validate the detected human activities.

2. The device of claim 1, wherein
the motion sensor (12; 26) is configured to generate a motion measurement signal that indicates measured motions;
the processing unit (16; 28) is configured to perform a human activity recognition based on the motion measurement signal generated by the motion sensor (12);
the electrostatic charge sensor (14; 32) is configured to measure an electrostatic charge of a surrounding environment, and to generate an electrostatic charge measurement signal that indicates the measured electrostatic charges;
the processing unit (16; 36) is configured to perform a context detection, detecting whether the device (10) is in an indoor or outdoor environment based on the electrostatic charge signal generated by electrostatic charge sensor (14; 32), using either a frequency approach or a time domain approach; and
the processing unit (16; 36) is configured to validate the detected human activity by rejecting false state detections.

3. The device of claim 2, wherein the processing unit (16) determines the device (10) is indoors in a case where the electrostatic charge measurement includes a first peak at a frequency corresponding to an alternating current (AC) power line.

4. The device of claim 3, wherein the processing unit (16) determines the device (10) is outdoors in a case where the electrostatic charge measurement does not include a peak at the frequency corresponding to the AC power line, or the electrostatic charge measurement includes a second peak, which is smaller than the first peak, at the frequency corresponding to the AC power line.

5. The device of claim 2, wherein the processing unit (16) is configured to determine at least one feature of the electrostatic charge measurement, and determine whether the device (10) is indoors or outdoors based on the at least one feature.

6. The device of claim 5, wherein the at least one feature includes at least one of an energy calculation, a variance calculation, a zero crossing calculation, a peak-to-peak calculation, a peak count calculation, an absolute mean calculation, a maximum calculation, or a minimum calculation.

7. The device of any of the preceding claims, wherein the activity state is a state selected from a group of states including a stationary state that indicates the user is remaining still, an indoor walking state that indicates the user is walking indoors, an outdoor walking state that indicates the user is walking outside, a stationary bicycle state that indicates the user is cycling on a stationary bicycle, an outdoor cycling state that indicates the user is cycling outdoors, and a driving state that indicates the user is driving a vehicle.

8. The device of any of the preceding claims, wherein the electrostatic charge sensor (12) includes a single electrode or a plurality of electrodes.

9. The device of any of the preceding claims, wherein the driving state indicates that the user is driving a vehicle, and the driving state is invalidated in response to determining the device is indoors.

10. The device according to any of the preceding claims, wherein the processing unit (16) is configured to determine a first activity state of a user of the device based on the motion measurement, switch the first activity state to a second activity state based on the electrostatic charge measurement, and output the second activity state.

11. The device of the preceding claim, wherein,
in a case where the first activity state is a walking state that indicates the user is walking and the processing unit determines the device is indoors, the second activity state is indoor walking state that indicates the user is walking indoors, and,
in a case where the first activity state is the walking state and the processing unit determines the device is outdoors, the second activity state is an outdoor walking state that indicates the user is walking outside.

12. The device of claim 10 or 11, wherein
in a case where the first activity state is a cycling state that indicates the user is cycling and the processing unit determines the device is indoors, the second activity state is a stationary bicycle state that indicates the user is cycling on a stationary bicycle, and,
in a case where the first activity state is the cycling state and the processing unit determines the device is outdoors, the second activity state is an outdoor cycling state that indicates the user is cycling outdoors.

13. A method for detecting human activity using a device including a motion sensor (12), and an electrostatic charge sensor (14), the method comprising:
generating a motion measurement of the device using the motion sensor (12), the motion measurement detecting human activities, such as remaining stationary, walking, cycling or driving;
generating an electrostatic charge measurement of the device using the electrostatic charge sensor (14), the electrostatic charge measurement detecting whether the device is in an indoor or outdoor environment;
determining an activity state of a user of the device based on the motion measurement and the electrostatic charge measurement; and
outputting the activity state, **characterised in that** the determination of the activity state includes using the detected indoor or outdoor environment in conjunction with the detected human activity to validate the detected human activities.

14. The method of the preceding claim, wherein:
generating a motion measurement of the device comprises generating a motion measurement signal that indicates measured motions;
generating an electrostatic charge measurement comprises measuring an electrostatic charge of a surrounding environment, and generating an electrostatic charge measurement signal that indicates the measured electrostatic charges; and
determining an activity state of a user comprises performing a human activity recognition based on the motion measurement signal generated by the motion sensor (12); performing a context detection, detecting whether the device (10) is in an indoor or outdoor environment based on the electrostatic charge signal generated by electrostatic charge sensor (14; 32) using either a frequency approach or a time domain approach; and validating the detected human activity by rejecting false state detections.

15. The method of claim 13 or 14, comprising:
determining the device (10) is indoors in a case where the electrostatic charge measurement includes a first peak at a frequency corresponding to an alternating current (AC) power line and determining the device (10) is outdoors in a case where the electrostatic charge measurement does not include a peak at the frequency corresponding to the AC power line, or the electrostatic charge measurement includes a second peak, which is smaller than the first peak, at the frequency corresponding to the AC power line; or determining at least one feature of the electrostatic charge measurement, and determining whether the device (10) is indoors or outdoors based on the at least one feature, wherein the at least one feature includes at least one of an energy calculation, a variance calculation, a zero crossing calculation, a peak-to-peak calculation, a peak count calculation, an absolute mean calculation, a maximum calculation, or a minimum calculation.

## Patentansprüche

1. Vorrichtung zum Detektieren menschlicher Aktivitäten, die Folgendes umfasst:
einen Bewegungssensor (12), der dazu konfiguriert ist, eine Bewegungsmessung der Vorrichtung zu erzeugen, wobei die Bewegungsmessung menschliche Aktivitäten detektiert, wie etwa Stehenbleiben, Gehen, Radfahren oder Fahren;
einen elektrostatischen Ladungssensor (14), der dazu konfiguriert ist, eine elektrostatische Ladungsmessung der Vorrichtung zu erzeugen, wobei die elektrostatische Ladungsmessung detektiert, ob sich die Vorrichtung in einer Innen- oder Außenumgebung befindet; und
eine Verarbeitungseinheit (16), die dazu konfiguriert ist, einen Aktivitätsstatus eines Benutzers der Vorrichtung auf Basis der Bewegungsmessung und der elektrostatischen Ladungsmessung zu bestimmen und den Aktivitätszustand auszugeben, **dadurch gekennzeichnet, dass** die Verarbeitungseinheit dazu konfiguriert ist, die detektierte Innen- oder Außenumgebung in Verbindung mit der detektierten menschlichen Aktivität zu verwenden, um die detektierten menschlichen Aktivitäten zu überprüfen.

2. Vorrichtung nach Anspruch 1, wobei
der Bewegungssensor (12; 26) dazu konfiguriert ist, ein Bewegungsmesssignal zu erzeugen, das gemessene Bewegungen angibt;
die Verarbeitungseinheit (16; 28) dazu konfiguriert ist, eine menschliche Aktivitätserkennung auf Basis des durch den Bewegungssensor (12) erzeugten Bewegungsmesssignals durchzuführen;
der elektrostatische Ladungssensor (14; 32) dazu konfiguriert ist, eine elektrostatische Ladung einer umliegenden Umgebung zu messen und ein elektrostatisches Ladungsmesssignal zu erzeugen, das die gemessenen elektrostatischen Ladungen angibt;
die Verarbeitungseinheit (16; 36) dazu konfiguriert ist, eine Kontextdetektion durchzuführen, die auf Basis des durch den elektrostatischen Ladungssensor (14; 32) erzeugten elektrostatischen Ladungssignals unter Verwendung entweder eines Frequenzansatzes oder eines Zeitbereichsansatzes detektiert, ob sich die Vorrichtung (10) in einer Innen- oder Außenumgebung befindet; und die Verarbeitungseinheit (16; 36) dazu konfiguriert ist, die detektierte menschliche Aktivität zu überprüfen, indem sie falsche Zustandsdetektionen zurückweist.

3. Vorrichtung nach Anspruch 2, wobei die Verarbeitungseinheit (16) bestimmt, dass sich die Vorrichtung (10) in einem Innenbereich befindet, wenn die elektrostatische Ladungsmessung einen ersten Spitzenwert bei einer Frequenz enthält, die einer Wechselstromleitung (AC-Leitung) entspricht.

4. Vorrichtung nach Anspruch 3, wobei die Verarbeitungseinheit (16) bestimmt, dass sich die Vorrichtung (10) in einem Außenbereich befindet, wenn die elektrostatische Ladungsmessung keinen Spitzenwert bei der Frequenz enthält, die der AC-Leitung entspricht, oder die elektrostatische Ladungsmessung einen zweiten Spitzenwert, der kleiner als der erste Spitzenwert ist, bei der Frequenz enthält, die der AC-Leitung entspricht.

5. Vorrichtung nach Anspruch 2, wobei die Verarbeitungseinheit (16) dazu konfiguriert ist, mindestens ein Merkmal der elektrostatischen Ladungsmessung zu bestimmen und auf Basis des mindestens einen Merkmals zu bestimmen, ob sich die Vorrichtung (10) in einem Innen- oder einem Außenbereich befindet.

6. Vorrichtung nach Anspruch 5, wobei das mindestens eine Merkmal mindestens eine von einer Energieberechnung, einer Varianzberechnung, einer Nulldurchgangsberechnung, einer Spitze-zu-Spitze-Berechnung, einer Spitzenwertberechnung, einer Berechnung des absoluten Mittelwerts, einer Maximalberechnung oder einer Minimalberechnung enthält.

7. Vorrichtung nach einem der vorhergehenden Ansprüche, wobei der Aktivitätszustand ein Zustand ist, der aus einer Gruppe von Zuständen ausgewählt wird, einschließlich eines stationären Zustands, der angibt, dass der Benutzer still steht, eines Innenbereich-Gehzustands, der angibt, dass der Benutzer in einem Innenbereich geht, eines Außenbereich-Gehzustands, der angibt, dass der Benutzer in einem Außenbereich geht, eines Standfahrradzustands, der angibt, dass der Benutzer auf einem Standfahrrad fährt, eines Außenbereich-Radfahrzustand, der angibt, dass der Benutzer in einem Außenbereich mit dem Fahrrad fährt, und eines Fahrzustands, der angibt, dass der Benutzer ein Fahrzeug fährt.

8. Vorrichtung nach einem der vorhergehenden Ansprüche, wobei der elektrostatische Ladungssensor (14) eine einzelne Elektrode oder mehrere Elektroden enthält.

9. Vorrichtung nach einem der vorhergehenden Ansprüche, wobei der Fahrzustand angibt, dass der Benutzer ein Fahrzeug fährt, und der Fahrzustand als Reaktion auf das Bestimmen, dass sich die Vorrichtung in einem Innenbereich befindet, ungültig wird.

10. Vorrichtung nach einem der vorhergehenden Ansprüche, wobei die Verarbeitungseinheit (16) dazu konfiguriert ist, einen ersten Aktivitätszustand eines Benutzers der Vorrichtung auf Basis der Bewegungsmessung zu bestimmen, vom ersten Aktivitätszustand auf Basis der elektrostatischen Ladungsmessung zu einem zweiten Aktivitätszustand zu wechseln und den zweiten Aktivitätszustand auszugeben.

11. Vorrichtung nach dem vorhergehenden Anspruch, wobei, wenn der erste Aktivitätszustand ein Gehzustand ist, der angibt, dass der Benutzer geht, und die Verarbeitungseinheit bestimmt, dass sich die Vorrichtung in einem Innenbereich befindet, der zweite Aktivitätszustand ein Innenbereich-Gehzustand ist, der angibt, dass der Benutzer in einem Innenbereich geht, und, wenn der erste Aktivitätszustand der Gehzustand ist und die Verarbeitungseinheit bestimmt, dass sich die Vorrichtung in einem Außenbereich befindet, der zweite Aktivitätszustand ein Außenbereich-Gehzustand ist, der angibt, dass der Benutzer in einem Außenbereich geht.

12. Vorrichtung nach Anspruch 10 oder 11, wobei,
wenn der erste Aktivitätszustand ein Radfahrzustand ist, der angibt, dass der Benutzer Fahrrad fährt, und die Verarbeitungseinheit bestimmt, dass sich die Vorrichtung in einem Innenbereich befindet, der zweite Aktivitätszustand ein Standfahrradzustand ist, der angibt, dass der Benutzer auf einem Standfahrrad fährt, und,
wenn der erste Aktivitätszustand der Radfahrzustand ist und die Verarbeitungseinheit bestimmt, dass sich die Vorrichtung in einem Außenbereich befindet, der zweite Aktivitätszustand ein Außenbereich-Radfahrzustand ist, der angibt, dass der Benutzer in einem Außenbereich Fahrrad fährt.

13. Verfahren zum Detektieren menschlicher Aktivität unter Verwendung einer Vorrichtung, die einen Bewegungssensor (12) und einen elektrostatischen Ladungssensor (14) enthält, wobei das Verfahren Folgendes umfasst:
Erzeugen einer Bewegungsmessung der Vorrichtung unter Verwendung des Bewegungssensors (12), wobei die Bewegungsmessung menschliche Aktivitäten detektiert, wie etwa Stehenbleiben, Gehen, Radfahren oder Fahren;
Erzeugen einer elektrostatischen Ladungsmessung der Vorrichtung unter Verwendung des elektrostatischen Ladungssensors (14), wobei die elektrostatische Ladungsmessung detektiert, ob sich die Vorrichtung in einer Innen- oder Außenumgebung befindet;
Bestimmen eines Aktivitätszustands eines Benutzers der Vorrichtung auf Basis der Bewegungsmessung und der elektrostatischen Ladungsmessung; und
Ausgeben des Aktivitätszustands, **dadurch gekennzeichnet, dass** das Bestimmen des Aktivitätszustands das Verwenden der detektierten Innen- oder Außenumgebung in Verbindung mit der detektierten menschlichen Aktivität umfasst, um die detektierten menschlichen Aktivitäten zu überprüfen.

14. Verfahren nach dem vorhergehenden Anspruch, wobei:
das Erzeugen einer Bewegungsmessung der Vorrichtung das Erzeugen eines Bewegungsmesssignals umfasst, das gemessene Bewegungen angibt;
das Erzeugen einer elektrostatischen Ladungsmessung das Messen einer elektrostatischen Ladung einer umliegenden Umgebung und das Erzeugen eines elektrostatischen Ladungsmesssignals, das die gemessenen elektrostatischen Ladungen angibt, umfasst; und
das Bestimmen eines Aktivitätszustands eines Benutzers das Durchführen einer menschlichen Aktivitätserkennung auf Basis des durch den Bewegungssensor (12) erzeugten Bewegungsmesssignals; das Durchführen einer Kontextdetektion, die auf Basis des durch den elektrostatischen Ladungssensor (14; 32) erzeugten elektrostatischen Ladungssignals unter Verwendung entweder eines Frequenzansatzes oder eines Zeitbereichsansatzes detektiert, ob sich die Vorrichtung (10) in einer Innen- oder Außenumgebung befindet; und das Überprüfen der detektierten menschlichen Aktivität durch Zurückweisen falscher Zustandsdetektionen umfasst.

15. Verfahren nach Anspruch 13 oder 14, das Folgendes umfasst:
Bestimmen, dass sich die Vorrichtung (10) in einem Innenbereich befindet, wenn die elektrostatische Ladungsmessung einen ersten Spitzenwert bei einer Frequenz enthält, die einer Wechselstromleitung (AC-Leitung) entspricht, und Bestimmen, dass sich die Vorrichtung (10) in einem Außenbereich befindet, wenn die elektrostatische Ladungsmessung keinen Spitzenwert bei der Frequenz enthält, die der AC-Leitung entspricht, oder die elektrostatische Ladungsmessung einen zweiten Spitzenwert, der kleiner als der erste Spitzenwert ist, bei der Frequenz enthält, die der AC-Leitung entspricht; oder Bestimmen mindestens eines Merkmals der elektrostatischen Ladungsmessung und Bestimmen auf Basis des mindestens einen Merkmals, ob sich die Vorrichtung (10) in einem Innen- oder einem Außenbereich befindet, wobei das mindestens eine Merkmal mindestens eine von einer Energieberechnung, einer Varianzberechnung, einer Nulldurchgangsberechnung, einer Spitze-zu-Spitze-Berechnung, einer Spitzenwertberechnung, einer Berechnung des absoluten Mittelwerts, einer Maximalberechnung oder einer Minimalberechnung enthält.

## Revendications

1. Dispositif de détection d'activité humaine, comprenant :
un capteur de mouvement (12) configuré pour produire une mesure de mouvement du dispositif, la mesure de mouvement détectant des activités humaines, comme la station immobile, la marche, le déplacement à bicyclette ou la conduite d'un véhicule ;
un capteur de charge électrostatique (14) configuré pour produire une mesure de charge électrostatique du dispositif, la mesure de charge électrostatique détectant si le dispositif est dans un environnement intérieur ou extérieur ; et
une unité de traitement (16) configurée pour déterminer un état d'activité d'un utilisateur du dispositif en se basant sur la mesure de mouvement et sur la mesure de charge électrostatique, et pour délivrer en sortie cet état d'activité, **caractérisé en ce que** l'unité de traitement est configurée pour utiliser l'environnement intérieur ou extérieur détecté en association avec l'activité humaine détectée pour valider les activités humaines détectées.

2. Dispositif selon la revendication 1, dans lequel :
le capteur de mouvement (12 ; 26) est configuré pour produire un signal de mesure de mouvement qui indique les mouvements mesurés ;
l'unité de traitement (16 ; 28) est configurée pour réaliser une reconnaissance d'activité humaine basée sur le signal de mesure de mouvement produit par le capteur de mouvement (12) ;
le capteur de charge électrostatique (14 ; 32) est configuré pour mesurer une charge électrostatique d'un environnement ambiant, et pour produire un signal de mesure de charge électrostatique qui indique les charges électrostatiques mesurées ;
l'unité de traitement (16 ; 36) est configurée pour réaliser une détection de contexte, détectant si le dispositif (10) est dans un environnement intérieur ou extérieur d'après le signal de charge électrostatique produit par le capteur de charge électrostatique (14 ; 32), en utilisant soit une approche fréquentielle, soit une approche dans le domaine temporel ; et
l'unité de traitement (16 ; 36) est configurée pour valider l'activité humaine détectée en rejetant les fausses détections d'état.

3. Dispositif selon la revendication 2, dans lequel l'unité de traitement (16) détermine que le dispositif (10) est en intérieur dans le cas où la mesure de charge électrostatique comporte une première crête à une fréquence correspondant à une ligne d'alimentation en courant alternatif, ou ligne c. a.

4. Dispositif selon la revendication 3, dans lequel l'unité de traitement (16) détermine que le dispositif (10) est en extérieur dans le cas où la mesure de charge électrostatique ne comporte pas de crête à la fréquence correspondant à la ligne c. a., ou si la mesure de charge électrostatique comporte une deuxième crête, qui est plus petite que la première crête, à la fréquence correspondant à la ligne c. a.

5. Dispositif selon la revendication 2, dans lequel l'unité de traitement (16) est configurée pour déterminer au moins une caractéristique de la mesure de charge électrostatique, et pour déterminer si le dispositif (10) est en intérieur ou en extérieur en se basant sur ladite au moins une caractéristique.

6. Dispositif selon la revendication 5, dans lequel ladite au moins une caractéristique comprend au moins un élément parmi un calcul d'énergie, un calcul de variance, un calcul de passage par zéro, un calcul crête-à-crête, un calcul de comptage de crêtes, un calcul de moyenne absolue, un calcul de maximum et un calcul de minimum.

7. Dispositif selon l'une quelconque des revendications précédentes, dans lequel l'état d'activité est un état choisi dans un groupe d'états comprenant un état statique qui indique que l'utilisateur reste immobile, un état de marche en intérieur qui indique que l'utilisateur marche en intérieur, un état de marche en extérieur qui indique que l'utilisateur marche en extérieur, un état de vélo fixe qui indique que l'utilisateur pédale sur un vélo fixe, un état de déplacement à bicyclette en extérieur qui indique que l'utilisateur se déplace à bicyclette en extérieur, et un état de conduite qui indique que l'utilisateur conduit un véhicule.

8. Dispositif selon l'une quelconque des revendications précédentes, dans lequel le capteur de charge électrostatique (14) comprend une seule électrode ou plusieurs électrodes.

9. Dispositif selon l'une quelconque des revendications précédentes, dans lequel l'état de conduite indique que l'utilisateur conduit un véhicule, et l'état de conduite est invalidé s'il est déterminé que le dispositif se trouve en intérieur.

10. Dispositif selon l'une quelconque des revendications précédentes, dans lequel l'unité de traitement (16) est configurée pour déterminer un premier état d'activité d'un utilisateur du dispositif en se basant sur la mesure de mouvement, faire passer le premier état d'activité à un deuxième état d'activité en se basant sur la mesure de charge électrostatique, et délivrer en sortie le deuxième état d'activité.

11. Dispositif selon la revendication précédente, dans lequel :
dans le cas où le premier état d'activité est un état de marche qui indique que l'utilisateur marche et où l'unité de traitement détermine que le dispositif est en intérieur, le deuxième état d'activité est un état de marche en intérieur qui indique que l'utilisateur marche en intérieur, et
dans le cas où le premier état d'activité est l'état de marche et où l'unité de traitement détermine que le dispositif est en extérieur, le deuxième état d'activité est un état de marche en extérieur qui indique que l'utilisateur marche en extérieur.

12. Dispositif selon la revendication 10 ou 11, dans lequel :
dans le cas où le premier état d'activité est un état de pédalage qui indique que l'utilisateur pédale et où l'unité de traitement détermine que le dispositif est en intérieur, le deuxième état d'activité est un état de vélo fixe qui indique que l'utilisateur pédale sur un vélo fixe, et
dans le cas où le premier état d'activité est l'état de pédalage et où l'unité de traitement détermine que le dispositif est en extérieur, le deuxième état d'activité est un état de déplacement à bicyclette en extérieur qui indique que l'utilisateur se déplace à bicyclette en extérieur.

13. Procédé de détection d'activité humaine utilisant un dispositif qui comporte un capteur de mouvement (12) et un capteur de charge électrostatique (14), le procédé comprenant les étapes suivantes :
produire une mesure de mouvement du dispositif en utilisant le capteur de mouvement (12), la mesure de mouvement détectant des activités humaines, comme la station immobile, la marche, le déplacement à bicyclette ou la conduite d'un véhicule ;
produire une mesure de charge électrostatique du dispositif en utilisant le capteur de charge électrostatique (14), la mesure de charge électrostatique détectant si le dispositif est dans un environnement intérieur ou extérieur ;
déterminer un état d'activité d'un utilisateur du dispositif en se basant sur la mesure de mouvement et sur la mesure de charge électrostatique ; et
délivrer en sortie l'état d'activité, **caractérisé en ce que** la détermination de l'état d'activité comprend le fait d'utiliser l'environnement intérieur ou extérieur détecté en association avec l'activité humaine détectée pour valider les activités humaines détectées.

14. Procédé selon la revendication précédente, dans lequel :
la production d'une mesure de mouvement du dispositif comprend la production d'un signal de mesure de mouvement qui indique les mouvements mesurés ;
la production d'une mesure de charge électrostatique comprend la mesure d'une charge électrostatique d'un environnement ambiant, et la production d'un signal de mesure de charge électrostatique qui indique les charges électrostatiques mesurées ; et
la détermination d'un état d'activité d'un utilisateur comprend la réalisation d'une reconnaissance d'activité humaine basée sur le signal de mesure de mouvement produit par le capteur de mouvement (12) ; la réalisation d'une détection de contexte, détectant si le dispositif (10) est dans un environnement intérieur ou extérieur d'après le signal de charge électrostatique produit par le capteur de charge électrostatique (14 ; 32), en utilisant soit une approche fréquentielle, soit une approche dans le domaine temporel ; et la validation de l'activité humaine détectée en rejetant les fausses détections d'état.

15. Procédé selon la revendication 13 ou 14, comprenant les opérations suivantes :
déterminer que le dispositif (10) est en intérieur dans le cas où la mesure de charge électrostatique comporte une première crête à une fréquence correspondant à une ligne d'alimentation en courant alternatif, ou ligne c. a., et déterminer que le dispositif (10) est en extérieur dans le cas où la mesure de charge électrostatique ne comporte pas de crête à la fréquence correspondant à la ligne c. a., ou si la mesure de charge électrostatique comporte une deuxième crête, qui est plus petite que la première crête, à la fréquence correspondant à la ligne c. a. ; ou déterminer au moins une caractéristique de la mesure de charge électrostatique, et déterminer si le dispositif (10) est en intérieur ou en extérieur en se basant sur ladite au moins une caractéristique, dans lequel ladite au moins une caractéristique comprend au moins un élément parmi un calcul d'énergie, un calcul de variance, un calcul de passage par zéro, un calcul crête-à-crête, un calcul de comptage de crêtes, un calcul de moyenne absolue, un calcul de maximum et un calcul de minimum.
